(19) Europäisches Patentamt
European Patent Office
Office européen des brevets

(11) Veröffentlichungsnummer: **0 297 393**
**A2**

(12) # EUROPÄISCHE PATENTANMELDUNG

(21) Anmeldenummer: 88109831.3

(51) Int. Cl.4: C07D 249/08 , A01N 43/653

(22) Anmeldetag: 21.06.88

(30) Priorität: 01.07.87 DE 3721695

(43) Veröffentlichungstag der Anmeldung: 04.01.89 Patentblatt 89/01

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Kraatz, Udo Dr
Andreasstr 22a
D 5090 Leverkusen 1(DE)
Erfinder: Lürssen, Klaus, Dr.
August-Kierspel-Strasse 145
D 5060 Bergisch-Gladbach 2(DE)

(54) **1-Phenoxy-3-triazolyl-1-hexen-Derivate.**

(57) Neue 1-Phenoxy-3-triazolyl-1-hexen-Derivate der Formel

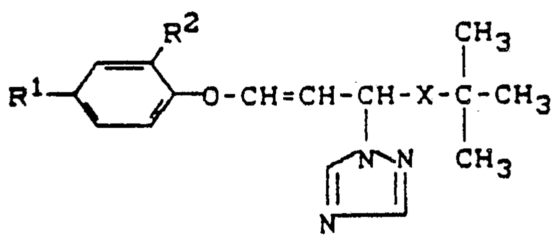

(I)

in welcher
$R^1$ und $R^2$ unabhängig voneinander für Wasserstoff, Halogen, Alkyl mit 1 bis 4 Kohlenstoffatomen, Alkoxy und Alkylthio mit jeweils 1 bis 4 Kohlenstoffatomen; Halogenalkyl mit 1 oder 2 Kohlenstoffatomen und 1 bis 5 Halogenatomen, Halogenalkoxy und Halogenalkylthio mit jeweils 1 oder 2 Kohlenstoffatomen und 1 bis 5 Halogenatomen stehen und
X für die Ketogruppe oder die CH(OH)-Gruppe steht,
sowie deren Säureadditions-Salze und Metallsalz-Komplexe, mehrere Verfahren zur Herstellung der neuen Stoffe und deren Verwendung als Pflanzenwachstumsregulatoren.

EP 0 297 393 A2

# 1-Phenoxy-3-triazolyl-1-hexen-Derivate

Die vorliegende Erfindung betrifft neue 1-Phenoxy-3-triazolyl-1-hexen-Derivate, mehrere Verfahren zu ihrer Herstellung sowie ihre Verwendung als Pflanzenwachstumsregulatoren.

Es ist bereits bekannt, daß zahlreiche im Phenylteil substituierte 1-Phenyl-2-azolyl-Derivate pflanzenwachstumsregulierende Eigenschaften besitzen (vgl. EP-OS 0 015 387 und US-PS 4 203 995). So lassen sich z.B. 1-(2,4-Dichlorphenyl)-4-ethyl-2-(1,2,4-triazol-1-yl)-2-hexen-1-ol, 1-(2,4-Dichlorphenyl)-4-methyl-2-(1,2,4-triazol-1-yl)-2-hepten-1-ol, 1-(2,4-Dichlorphenyl)-4-methyl-2-(1,2,4-triazol-1-yl)-2-hexen-1-ol und 1-(2,4-Dichlorphenyl)-2-(1,2,4-triazol-1-yl)-4,4-dimethyl-1-penten-3-ol zur Regulierung des Pflanzenwachstums einsetzen. Allerdings ist die Wirksamkeit dieser Stoffe, vor allem bei niedrigen Aufwandmengen, nicht immer ausreichend.

Es wurden nun neue 1-Phenoxy-3-triazolyl-1-hexen-Derivate der Formel

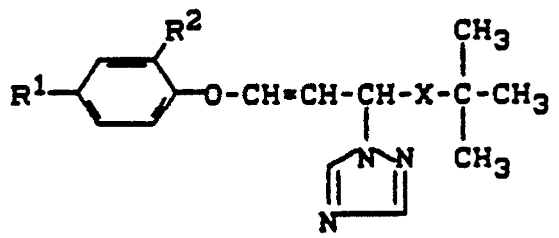

(I)

in welcher

$R^1$ und $R^2$ unabhängig voneinander für Wasserstoff, Halogen, Alkyl mit 1 bis 4 Kohlenstoffatomen, Alkoxy und Alkylthio mit jeweils 1 bis 4 Kohlenstoffatomen; Halogenalkyl mit 1 oder 2 Kohlenstoffatomen und 1 bis 5 Halogenatomen, Halogenalkoxy und Halogenalkylthio mit jeweils 1 oder 2 Kohlenstoffatomen und 1 bis 5 Halogenatomen stehen und

X für die Ketogruppe oder die CH(OH)-Gruppe steht

sowie deren Säureadditions-Salze und Metallsalz-Komplexe gefunden.

Die Verbindungen der Formel (I) können in zwei geometrischen Isomerenformen vorliegen, je nach Anordnung der Gruppen, die an die Doppelbindung gebunden sind.

Die Verbindungen der Formel (I), in denen X für die CH(OH)-Gruppe steht, besitzen zwei benachbarte asymmetrisch substituierte Kohlenstoffatome. Sie können deshalb in den beiden geometrischen Isomeren (threo- und erythro-Form) vorliegen, die in unterschiedlichen Mengenverhältnissen anfallen können. Die Verbindungen der Formel (I), in denen X für die Ketogruppe steht, besitzen ein asymmetrisch substituiertes Kohlenstoffatom. In beiden Fällen können die Verbindungen der Formel (I) in verschiedenen optischen Isomerenformen vorliegen, die in unterschiedlichenMengenverhältnissen anfallen können. Die Erfindung betrifft sowohl die einzelnen Isomeren als auch deren Gemische.

Weiterhin wurde gefunden, daß man 1-Phenoxy-3-triazolyl-1-hexen-Derivate der Formel (I) sowie deren Säureadditions-Salze und Metallsalz-Komplexe erhält, wenn man

a) entweder

α) 3,3-Dimethyl-1-(1,2,4-triazol-1-yl)-2-(trimethylsiloxy)-but-1-en der Formel

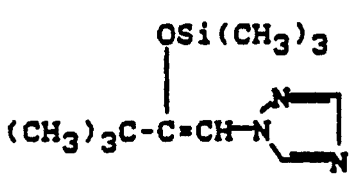

(II)

oder

β) 3,3-Dimethyl-1-(1,2,4-triazol-1-yl)-butan-2-on der Formel

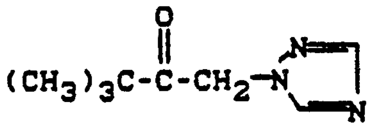

(IIa)

jeweils mit Aldehyden der Formel

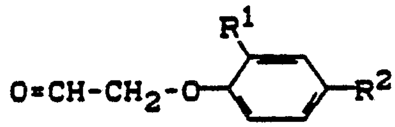

(III)

in welcher

$R^1$ und $R^2$ die oben angegebene Bedeutung haben,

in Gegenwart eines Verdünnungsmittels und in Gegenwart eines Katalysators umsetzt und von den sich bildenden Isomeren das gewünschte isomere Produkt der Formel

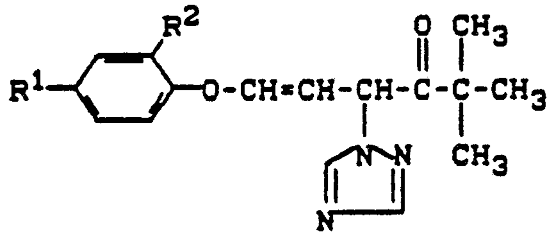

(Ia)

in welcher

$R^1$ und $R^2$ die oben angegebene Bedeutung haben,

abtrennt, oder

b) 1-Phenoxy-3-triazolyl-1-hexen-Derivate der Formel

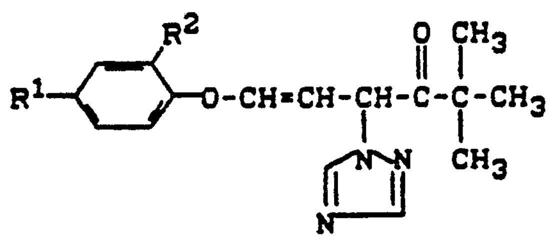

(Ia)

in welcher

$R^1$ und $R^2$ die oben angegebene Bedeutung haben,

entweder

α) mit komplexen Hydriden in Gegenwart eines Verdünnungsmittels umsetzt oder

β) mit Aluminiumisopropylat in Gegenwart eines Verdünnungsmittels umsetzt, und gegebenenfalls anschließend an die so erhaltenen 1-Phenoxy-3-triazolyl-1-hexen-Derivate der Formel (I) eine Säure oder ein Metallsalz addiert.

Schließlich wurde gefunden, daß die neuen 1-Phenoxy-3-triazolyl-1-hexen-Derivate der Formel (I) sowie deren Säureadditions-Salze und Metallsalz-Komplexe starke pflanzenwachstumsregulierende Eigenschafen besitzen.

Überraschenderweise zeigen die erfindungsgemäßen 1-Phenoxy-3-triazolyl-1-hexen-Derivate sowie deren Säureadditions-Salze und Metallsalz-Komplexe eine bessere pflanzenwachstumsregulierende Wirkung als 1-(2,4-Dichlorphenyl)-4-ethyl-2-(1,2,4-triazol-1-yl)-2-hexen-1-ol, 1-(2,4-Dichlorphenyl)-4-methyl-2-(1,2,4-triazol-1-yl)-2-hepten-1-ol, 1-(2,4-Dichlorphenyl)-4-methyl-2-(1,2,4-triazol-1-yl)-2-hexen-1-ol und 1-(2,4-Dichlorphenyl)-2-(1,2,4-triazol-1-yl)-4,4-dimethyl-1-penten-3-ol, welches konstitutionell ähnliche, vorbekannte Wirkstoffe gleicher Wirkungsrichtung sind.

Die erfindungsgemäßen 1-Phenoxy-3-triazolyl-1-hexen-Derivate sind durch die Formel (I) allgemein definiert. Bevorzugt sind diejenigen Verbindungen der Formel (I), in denen
$R^1$ für Wasserstoff, Fluor, Chlor, Brom, Methyl, Ethyl, n-Propyl, iso-Propyl, Methoxy, Ethoxy, Methylthio, Ethylthio, Trifluormethyl, Trifluormethoxy, Trifluormethylthio steht,
$R^2$ für Wasserstoff, Fluor, Chlor, Brom, Methyl, Ethyl, n-Propyl, iso-Propyl, Methoxy, Ethoxy, Methylthio, Ethylthio, Trifluormethyl, Trifluormethoxy, Trifluormethylthio steht und
X für die Ketogruppe oder die CH(OH)-Gruppierung steht.

Bevorzugte erfindungsgemäße Verbindungen sind auch Additionsprodukte aus Säuren und denjenigen 1-Phenoxy-3-triazolyl-1-hexen-Derivaten der Formel (I), in denen $R^1$, $R^2$ und X diejenigen Bedeutungen haben, die bereits im Zusammenhang mit der Beschreibung der erfindungsgemäßen Stoffe für diese Substituenten als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin und Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensymstems der Elemente und denjenigen 1-Phenoxy-3-triazolyl-Derivaten der Formel (I), in denen $R^1$, $R^2$ und X diejenigen Bedeutungen haben, die bereits im Zusammenhang mit der Beschreibung der erfindungsgemäßen Stoffe für diese Substituenten als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenphysiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Verbindungen seien die in der folgenden Tabelle 1 aufgeführten Stoffe genannt:

**Tabelle 1:**

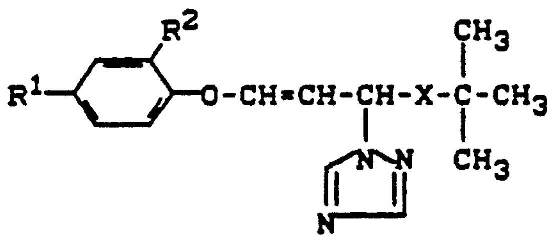

(I)

| $R^1$ | $R^2$ | X |
|---|---|---|
| Cl | H | CO |
| Cl | Cl | CO |
| Cl | $CH_3$ | CO |
| F | H | CO |
| $C_2H_5$ | H | CO |
| $OCH_3$ | H | CO |
| $SCH_3$ | H | CO |
| $CF_3$ | H | CO |
| $OCF_3$ | H | CO |
| $SCF_3$ | H | CO |
| H | Cl | CO |
| H | H | CO |
| Cl | H | >CH-OH |
| Cl | Cl | >CH-OH |
| Cl | $CH_3$ | >CH-OH |
| F | H | >CH-OH |
| $C_2H_5$ | H | >CH-OH |
| $OCH_3$ | H | >CH-OH |
| $SCH_3$ | H | >CH-OH |
| $CF_3$ | H | >CH-OH |

**Tabelle 1** (Fortsetzung)

| $R^1$ | $R^2$ | X |
|---|---|---|
| $OCF_3$ | H | >CH-OH |
| $SCF_3$ | H | >CH-OH |
| H | Cl | >CH-OH |
| H | H | >CH-OH |

Verwendet man 3,3-Dimethyl-1-(1,2,4-triazol-1-yl)-2-(trimethylsiloxy)-but-1-en und 4-Chlorphenoxyacetaldehyd als Ausgangssubstanzen und Tetrabutylammoniumfluorid als Katalysator, so kann der Verlauf des erfindungsgemäßen Verfahrens (a, Variante α) durch das folgende Formelschema wiedergegeben werden:

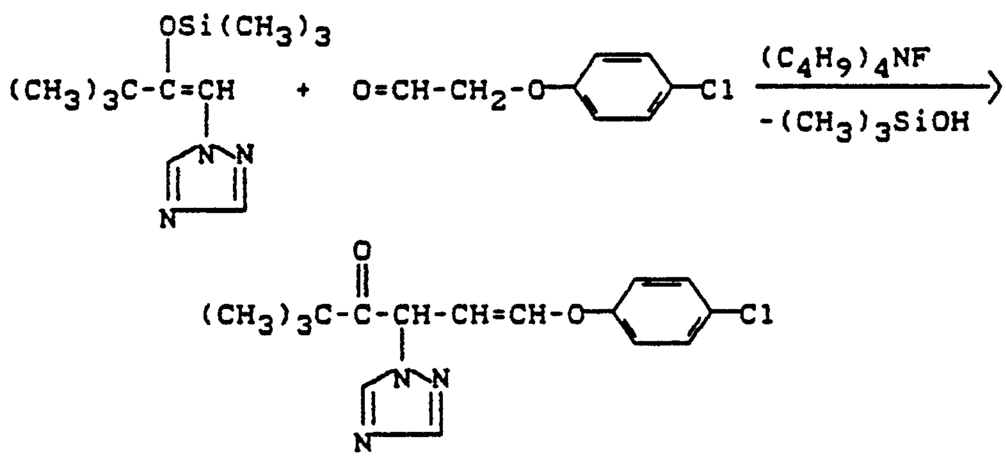


Verwendet man 3,3-Dimethyl-1-(1,2,4-triazol-1-yl)-butan-2-on und 4-Chlorphenoxy-acetaldehyd als Ausgangssubstanzen und Tetrabutylammoniumfluorid als Katalysator, so kann der Verlauf des erfindungsgemäßen Verfahrens (a, Variante β) durch das folgende Formelschema wiedergegeben werden:

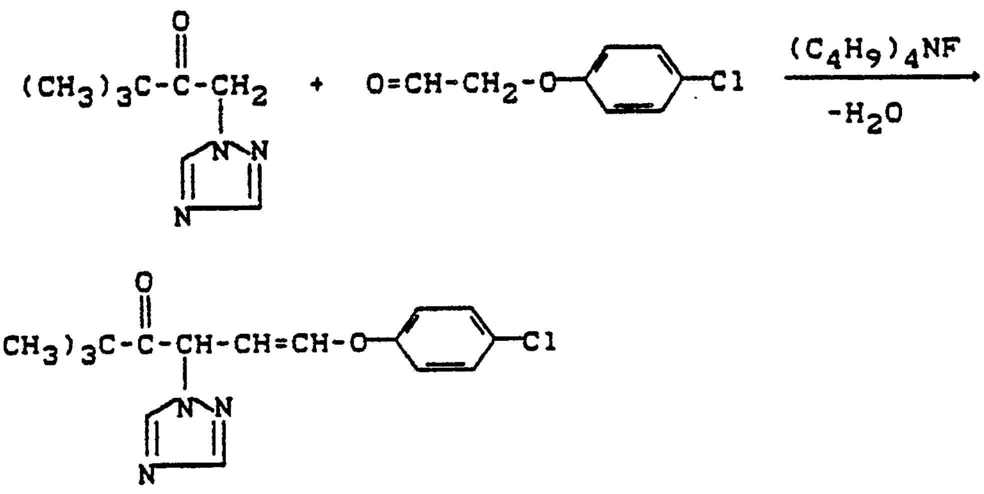


Verwendet man 5,5-Dimethyl-1-(4-chlorphenoxy)-3-(1,2,4-triazol-1-yl)-hex-1-en-4-on als Ausgangsstoff und Natriumborhydrid als Reduktionsmittel, so kann der Verlauf des erfindungsgemäßen Verfahrens (b, Variante α) durch das folgende Formelschema wiedergegeben werden:

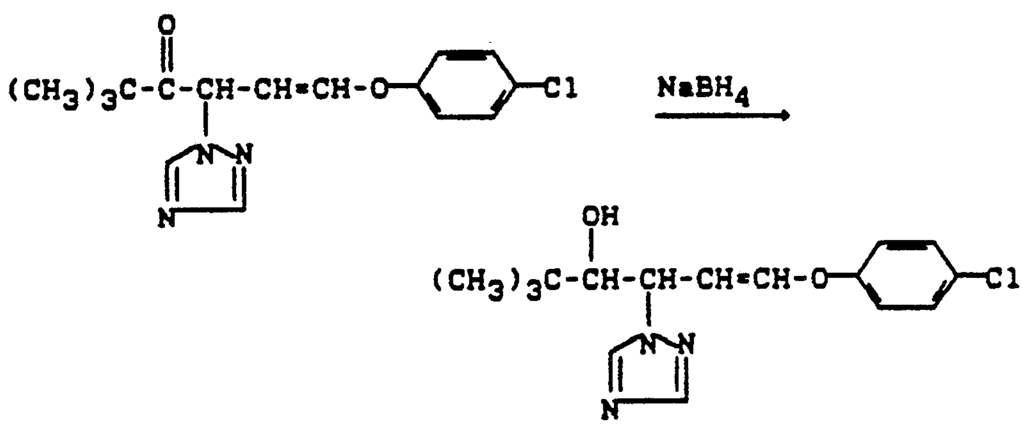


Verwendet man 5,5-Dimethyl-1-(4-chlorphenoxy)-3-(1,2,4-triazol-1-yl)-hex-1-en-4-on als Ausgangsstoff und Aluminiumisopropylat als Reduktionsmittel, so kann der Verlauf des erfindungsgemäßen Verfahrens (b, Variante β) durch das folgende Formelschema wiedergegeben werden:

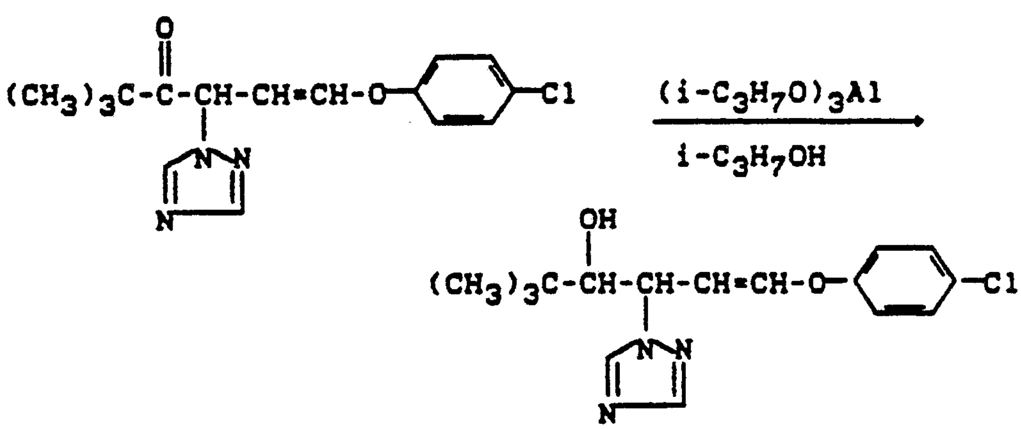


Das bei der Durchführung des erfindungsgemäßen Verfahrens (a, Variante α) als Ausgangsstoff zu verwendende 3,3-Dimethyl-1-(1,2,4-triazol-1-yl)-2-trimethylsiloxy)-but-1-en der Formel (II)ist bekannt (vgl.

DE-OS 35 25 978).

Das bei der Durchführung des erfindungsgemäßen Verfahrens (a, Variante β) als Ausgangsstoff zu verwendende 3,3-Dimethyl-1-(1,2,4-triazol-1-yl)-butan-2-on der Formel (IIa) ist ebenfalls bekannt (vgl. DE-OS 35 25 978).

Die bei der Durchführung des erfindungsgemäßen Verfahrens (a, Variante α und β) weiterhin als Ausgangsstoffe zu verwendenden Aldehyde sind durch die Formel (III) allgemein definiert. In dieser Formel haben $R^1$ und $R^2$ vorzugsweise die Bedeutungen, die bereits im Zusammenhang mit der Beschreibung der erfindungsgemäßen Stoffe der Formel (I) für diese Substituenten als bevorzugt genannt wurden.

Die Aldehyde der Formel (III) sind allgemein bekannte Verbindungen der organischen Chemie.

Als Verdünnungsmittel kommen für das erfindungsgemäße Verfahren (a) sowohl bei der Variante (α) als auch bei der Variante (β) alle üblichen inerten organischen Lösungsmittel infrage. Vorzugsweise verwendbar sind Alkohole, wie Methanol und Ethanol; Ether, wie Tetrahydrofuran und Dioxan; aliphatische und cycloaliphatische Kohlenwasserstoffe, wie Hexan und Cyclohexan; aromatische Kohlenwasserstoffe, wie Benzol, Toluol und Cumol; halogenierte aliphatische und aromatische Kohlenwasserstoffe, wie Methylenchlorid, Tetrachlorkohlenstoff, Chloroform, Chlorbenzol, Dichlorbenzol sowie Eisessig und Ameisensäure.

Bei der Durchführung des erfindungsgemäßen Verfahrens (a) kommen als Katalysatoren sowohl bei der Variante (α) als auch bei der Variante (β) alle für derartige Umsetzungen üblichen Reaktionsbeschleuniger in Betracht. Verwendbar sind saure oder basische Katalysatoren und Puffergemische aus sauren und basischen Substanzen. Besonders bevorzugt verwendbar sind Zinkchlorid, Titantetrachlorid, Eisen-III-chlorid, Lithiumfluorid, Tetraethylammoniumfluorid, Tetrabutylammoniumfluorid, Butyllithium, Natriumamid, Lithiumdiisopropylamid, Lithiumditrimethylsilylamid sowie Piperazin, Piperidin und β-Alanin.

Die Reaktionstemperaturen können bei der Durchführung des erfindungsgemäßen Verfahrens (a) sowohl beim Arbeiten nach der Variante (α) als auch nach der Variante (β) in einem größeren Bereich variiert werden. Im allgemeinen arbeitet man bei Temperaturen zwischen 10 °C und 100 °C, vorzugsweise bei Temperaturen zwischen 20 °C und 70 °C.

Bei der Durchführung des erfindungsgemäßen Verfahrens (a, Variante α) setzt man auf 1 Mol 3,3-Dimethyl-1-(1,2,4-triazol-1-yl)-2-(trimethylsiloxy)-but-1-en der Formel (II) 1 bis 1,5 Mol Aldehyd der Formel (III) und katalytische bis 0,2 molare Mengen an Katalysator ein.

Zur Isolierung der Verbindungen der Formel (Ia) werden die Reaktionsprodukte nach üblichen Methoden, wie z.B. chromatographisch, getrennt. Eine eindeutige Strukturzuordnung erfolgt aufgrund spektroskopischer Daten, insbesondere der NMR-Daten.

Bei der Durchführung des erfindungsgemäßen Verfahrens (a, Variante β) setzt man auf 1 Mol an 3,3-Dimethyl-1-(1,2,4-triazol-1-yl)-butan-2-on der Formel (IIa) 1 bis 1,5 Mol an Aldehyd der Formel (III) sowie katalytische bis 0,2 molare Mengen an Katalysator ein. Die Aufarbeitung erfolgt ebenso wie im Falle der Variante (α).

Die bei der Durchführung des erfindungsgemäßen Verfahrens (b) sowohl für die Variante (α) als auch für die Variante (β) als Ausgangsstoffe benötigten 1-Phenoxy-3-triazolyl-1-hexen-Derivate der Formel (Ia) sind erfindungsgemäße Stoffe. Sie lassen sich nach dem erfindungsgemäßen Verfahren (a) sowohl beim Arbeiten nach der Variante (α) als auch nach der Variante (β) herstellen.

Als komplexe Hydride kommen bei der Durchführung des Verfahrens (b, Variante α) alle üblichen zusammengesetzten Hydride in Frage. Vorzugsweise verwendbar sind Natriumborhydrid und Lithiumaluminiumhydrid.

Als Verdünnungsmittel kommen bei der Durchführung des erfindungsgemäßen Verfahrens (b, Variante α) alle für derartige Umsetzungen üblichen inerten organischen Solventien in Betracht. Vorzugsweise verwendbar sind Alkohole, wie Methanol, Ethanol, Butanol und Isopropanol, und außerdem Ether, wie Diethylether und Tetrahydrofuran.

Die Reaktionstemperaturen können bei der Durchführung des erfindungsgemäßen Verfahrens (b, Variante α) innerhalb eines bestimmten Bereiches variiert werden. Im allgemeinen arbeitet man bei Temperaturen zwischen 0 °C und 30 °C, vorzugsweise zwischen 0 °C und 20 °C.

Bei der Durchführung des erfindungsgemäßen Verfahrens (b, Variante α) setzt man auf 1 Mol an Keton der Formel (Ia) eine äquivalente Menge oder auch einen Überschuß an komplexen Hydriden ein. Zur Isolierung der gewünschten Verbindungen der Formel (I) verfährt man nach üblichen Methoden (vgl. Herstellungsbeispiele).

Bei der Durchführung des erfindungsgemäßen Verfahren (b, Variante β) dient Aluminiumisopropylat als Reduktionsmittel.

Als Verdünnungsmittel kommen bei der Durchführung des erfindungsgemäßen Verfahrens (b, Variante β) alle für derartige Umsetzungen üblichen inerten organischen Solventien in Frage. Vorzugsweise verwendbar sind Alkohole, wie Isopropanol, oder Kohlenwasserstoffe, wie Benzol.

Die Reaktionstemperaturen können bei der Durchführung des erfindungsgemäßen Verfahrens (b, Variante $\beta$) in einem größeren Bereich variiert werden. Im allgemeinen arbeitet man bei Temperaturen zwischen 20 °C und 120 °C, vorzugsweise zwischen 50 °C und 100 °C.

Bei der Durchführung des erfindungsgemäßen Verfahrens (b, Variante $\beta$) setzt man auf 1 Mol an Keton der Formel (Ia) 1 bis 2 Mol an Aluminiumisopropylat ein. Die Aufarbeitung erfolgt nach üblichen Methoden. Im allgemeinen geht man so vor, daß man das Reaktionsgemisch einengt, das verbleibende Produkt mit verdünnter, wäßriger Säure, wie Schwefelsäure, oder wäßriger Base, wie verdünnter wäßriger Natronlauge, behandelt, mit einem in Wasser wenig löslichen organischen Solvens extrahiert, die organische Phase trocknet und einengt.

Bei der Durchführung der erfindungsgemäßen Verfahren (a) und (b) arbeitet man im allgemeinen unter Normaldruck. Es ist jedoch auch möglich, unter erhöhtem oder vermindertem Druck zu arbeiten.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z. B. durch Lösen einer Verbindung der allgemeinen Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe von Verbindungen der allgemeinen Formel (I) können in einfacher Weise nach üblichen Verfahren erhalten werden, so z. B. durch Lösen des Metallsalzes in Alkohol, z. B. Ethanol, und Hinzufügen zu Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten-und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel

erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zukkerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall ver hindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlicht ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder - schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalo cyaninfarbstoffe und Spurennährstoffe, wie Salze von Eisen, Mangen, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe gehen aus den nachfolgenden Beispielen hervor.

## Herstellungsbeispiele:

### Beispiel 1

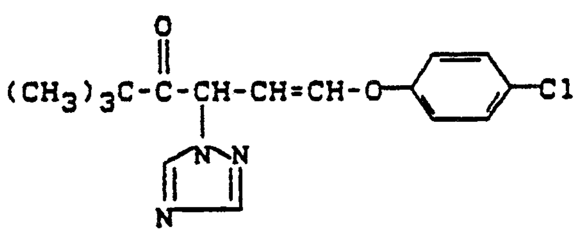

(Verfahren a, Variante α)

Zur Lösung von 78 g (0,326 Mol) 3,3-Dimethyl-1-(1,2,4-triazol-1-yl)-2-(trimethylsiloxy)-but-1-en und 55,6 g (0,326 Mol) 4-Chlorphenoxyacetaldehyd in 500 ml absolutem Tetrahydrofuran fügt man bei 20 °C unter Rühren 3 g Tetrabutylammoniumfluorid hinzu. Durch die leicht exotherme Reaktion steigt die Innentemperatur auf ca. 50 °C. Man rührt weitere 16 Stunden bei Raumtemperatur, verdünnt dann das Reaktionsgemisch mit Wasser und extrahiert mit Methylenchlorid. Die organische Phase wird unter vermindertem Druck eingeengt. Der Rückstand wird mit wenig Chlorform aufgenommen, wobei 24 g 5,5-Dimethyl-1-(4-chlorphenoxy)-2-hydroxy-3-(1,2,4-triazol-1-yl)-hexan-4-on auskristallisieren.

Das Filtrat wird an Kieselgel mit Chlorform/Essigester = 4:1 als Laufmittel chromatographiert.
Man erhält 23,8 g (23 % der Theorie) an 5,5-Dimethyl-1-(4-chlorphenoxy)-3-(1,2,4-triazol-1-yl)-hex-1-en-4-on vom Schmelzpunkt 106 °C.

## Beispiel 2

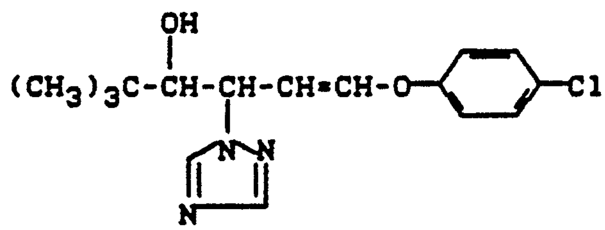

(Verfahren b, Variante α)

12 g (0,038 Mol) 5,5-Dimethyl-1-(4-chlorphenoxy)-3-(1,2,4-triazol-1-yl)-hex-1-en-4-on werden in 60 ml Methanol gelöst und bei Raumtemperatur mit 0,7 g (0,019 Mol) Natriumboranat versetzt. Man kocht 30 Minuten unter Rückfluß, gießt dann das Reaktionsgemisch in 150 ml Wasser und extrahiert mit Methylenchlorid. Die organische Phase wird zweimal mit Wasser gewaschen und unter vermindertem Druck eingeengt.
Man erhält 11,8 g (96,7 % der Theorie) an 5,5-Dimethyl-1-(4-chlorphenoxy)-4-hydroxy-3-(1,2,4-triazol-1-yl)-hex-1-en als Harz.

In analoger Weise zu den in den Beispielen 1 und 2 beschriebenen Methoden und unter Berücksichtigung der Angaben zu den erfindungsgemäßen Verfahren (a) und (b) werden die nachfolgend aufgeführten Verbindungen der Formel

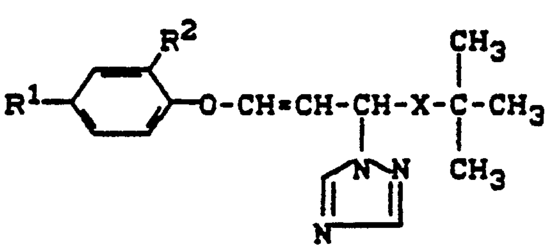 (I)

erhalten:

**Tabelle 2:**

| Bsp.Nr. | $R^1$ | $R^2$ | X | physikal. Konstante |
|---|---|---|---|---|
| 3 | Cl | Cl | CO | 90-93° C |
| 4 | Cl | Cl | $>$CH-OH | Harz |
| 5 | Cl | $CH_3$ | CO | Harz |
| 6 | Cl | $CH_3$ | $>$CH-OH | Harz |

Verwendungsbeispiele

In den folgenden Verwendungsbeispielen werden die nachstehend angegebenen Verbindungen als Vergleichssubstanzen eingesetzt:

(A)= 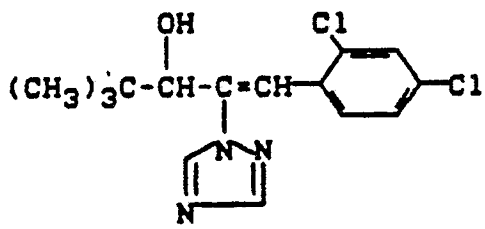

(bekannt aus US-PS 4 203 995)

(B)= 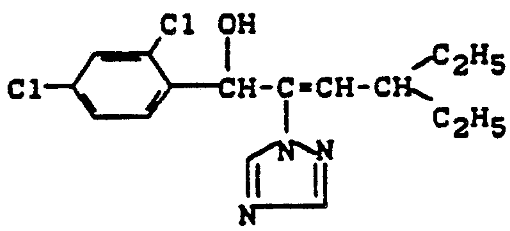

(C)= 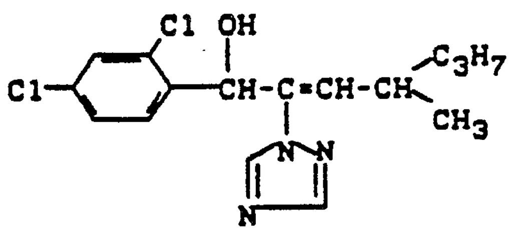

(D)= 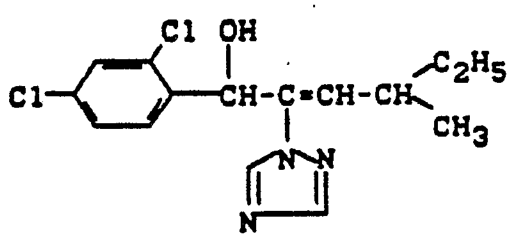

Die Verbindungen (B), (C) und (D) sind bekannt aus EP-OS 0 015 387.

Beispiel A

Wachstum bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 2 Wochen wird der Zuwachs der Pflanzen gemessen und das Wachstum in Prozent des Wachstums der Kontrollpflanzen berechnet. Es bedeuten 100 % Wachstum ein Wachstum entsprechend dem der Kontrollpflanzen und 0 % den Stillstand des Wachstums. Werte über 100 % kennzeichnen eine Wuchsförderung.

In diesem Test zeigt der erfindungsgemäße Wirkstoff (2) eine wesentlich stärkere Wuchshemmung als die Vergleichssubstanzen (A), (B) und (D).

Beispiel B

Wachstum bei Sojabohnen

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 2 Wochen wird bei allen Pflanzen der Zuwachs gemessen und das Wachstum in Prozent des Wachstums der Kontrollpflanzen berechnet. Es bedeuten 100 % Wachstum ein Wachstum entsprechend dem der Kontrollpflanzen und 0 % den Stillstand des Wachstums. Werte über 100 % kennzeichnen eine Wuchsförderung.

In diesem Test zeigt der erfindungsgemäße Wirkstoff (2) eine wesentlich stärkere Wuchshemmung als die Vergleichssubstanzen (B), (C) und (D).

## Ansprüche

1. 1-Phenoxy-3-triazolyl-1-hexen-Derivate der Formel

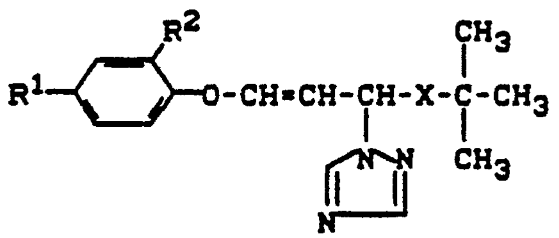

(I)

in welcher

$R^1$ und $R^2$ unabhängig voneinander für Wasserstoff, Halogen, Alkyl mit 1 bis 4 Kohlenstoffatomen, Alkoxy

und Alkylthio mit jeweils 1 bis 4 Kohlenstoffatomen; Halogenalkyl mit 1 oder 2 Kohlenstoffatomen und 1 bis 5 Halogenatomen, Halogenalkoxy und Halogenalkylthio mit jeweils 1 oder 2 Kohlenstoffatomen und 1 bis 5 Halogenatomen stehen und
X für die Ketogruppe oder die CH(OH)-Gruppe steht
sowie deren Säureadditions-Salze und Metallsalz-Komplexe.

2. Verbindungen der Formel (I) gemäß Anspruch 1, in denen
$R^1$ für Wasserstoff, Fluor, Chlor, Brom, Methyl, Ethyl, n-Propyl, iso-Propyl, Methoxy, Ethoxy, Methylthio, Ethylthio, Trifluormethyl, Trifluormethoxy, Trifluormethylthio steht,
$R^2$ für Wasserstoff, Fluor, Chlor, Brom, Methyl, Ethyl, n-Propyl, iso-Propyl, Methoxy, Ethoxy, Methylthio, Ethylthio, Trifluormethyl, Trifluormethoxy, Trifluormethylthio steht und
X für die Ketogruppe oder die CH(OH)-Gruppierung steht.

3. Verfahren zur Herstellung von 1-Phenoxy-3-triazolyl-1-hexen-Derivaten der Formel

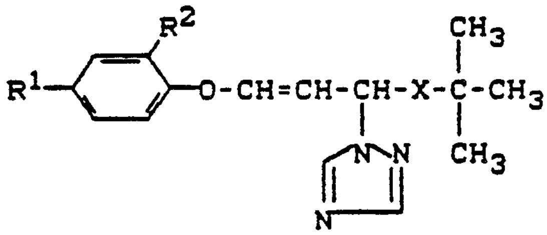
(I)

in welcher
$R^1$ und $R^2$ unabhängig voneinander für Wasserstoff, Halogen, Alkyl mit 1 bis 4 Kohlenstoffatomen, Alkoxy und Alkylthio mit jeweils 1 bis 4 Kohlenstoffatomen; Halogenalkyl mit 1 oder 2 Kohlenstoffatomen und 1 bis 5 Halogenatomen, Halogenalkoxy und Halogenalkylthio mit jeweils 1 oder 2 Kohlenstoffatomen und 1 bis 5 Halogenatomen stehen und
X für die Ketogruppe oder die CH(OH)-Gruppe steht,
sowie von deren Säureadditions-Salzen und Metallsalz-Komplexen, dadurch gekennzeichnet, daß man
a) entweder
α) 3,3-Dimethyl-1-(1,2,4-triazol-1-yl)-2-trimethylsiloxy)-but-1-en der Formel

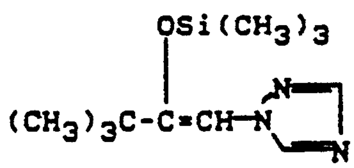
(II)

oder
β) 3,3-Dimethyl-1-(1,2,4-triazol-1-yl)-butan-2-on der Formel

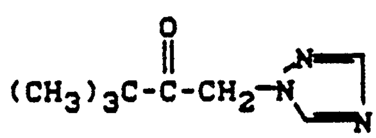
(IIa)

jeweils mit Aldehyden der Formel

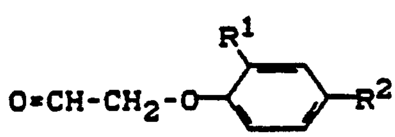
(III)

in welcher
$R^1$ und $R^2$ die oben angegebene Bedeutung haben,
in Gegenwart eines Verdünnungsmittels und in Gegenwart eines Katalysators umsetzt und von den sich

bildenden Isomeren das gewünschte isomere Produkt der Formel

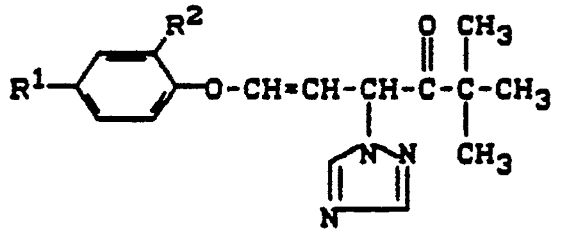 (Ia)

in welcher

$R^1$ und $R^2$ die oben angegebene Bedeutung haben,

abtrennt,

oder

b) 1-Phenoxy-3-triazolyl-1-hexen-Derivate der Formel

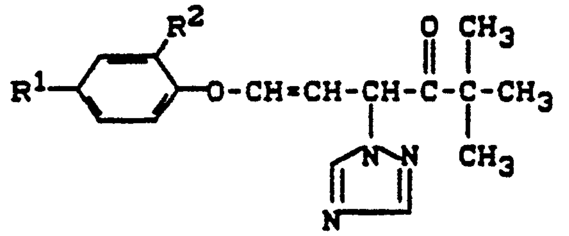 (Ia)

in welcher

$R^1$ und $R^2$ die oben angegebene Bedeutung haben,

entweder

α) mit komplexen Hydriden in Gegenwart eines Verdünnungsmittels umsetzt oder

β) mit Aluminiumisopropylat in Gegenwart eines Verdünnungsmittels umsetzt,

und gegebenenfalls anschließend an die so erhaltenen 1-Phenoxy-3-triazolyl-1-hexen-Derivate der Formel (I) eine Säure oder ein Metallsalz addiert.

4. Pflanzenwuchsregulierende Mittel, gekennzeichnet durch einen Gehalt an mindestens einem 1-Phenoxy-3-triazolyl-1-hexen-Derivat der Formel (I) gemäß Anspruch 1 bzw. an einem Säureadditionssalz oder Metallsalz-Komplex eines 1-Phenoxy-3-triazolyl-1-hexen-Derivates der Formel (I).

5. Verwendung von 1-Phenoxy-3-triazolyl-1-hexen-Derivaten der Formel (I) gemäß Anspruch 1 bzw. von deren Säureadditionssalzen oder Metallsalz-Komplexen zur Regulierung des Pflanzenwachstums.

6. Verfahren zur Regulierung des Pflanzenwachstums, dadurch gekennzeichnet, daß man 1-Phenoxy-3-triazolyl-1-hexen-Derivate der Formel (I) gemäß Anspruch 1 bzw. deren Säureadditionssalze oder Metallsalz-Komplexe auf die Pflanzen und/oder deren Lebensraum ausbringt.

7. Verfahren zur Herstellung von pflanzenwuchsregulierenden Mitteln, dadurch gekennzeichnet, daß man 1-Phenoxy-3-triazolyl-1-hexen-Derivate der Formel (I) gemäß Anspruch 1 bzw. deren Säureadditionssalze oder Metallsalz-Komplexe mit Streckmitteln und/oder oberflächenaktiven Stoffen vermischt.

8. 1-Phenoxy-3-triazolyl-1-hexen-Derivat gemäß Anspruch 1, gekennzeichnet durch die Formel

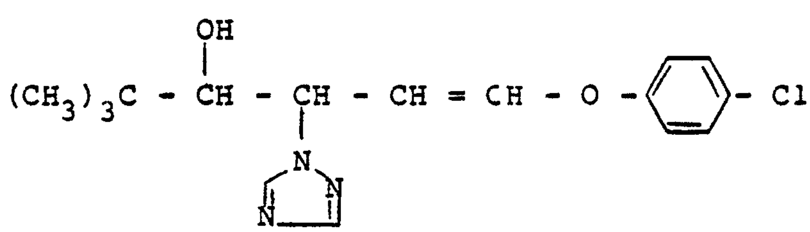

9. 1-Phenoxy-3-triazolyl-1-hexen-Derivat gemäß Anspruch 1, gekennzeichnet durch die Formel

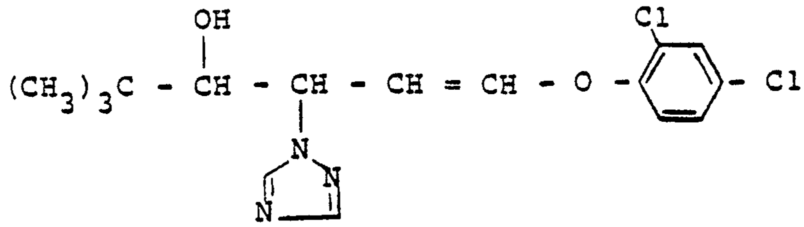

10. 1-Phenoxy-3-triazolyl-1-hexen-Derivate gemäß Anspruch 1. gekennzeichnet durch die Formel